# EUROPÄISCHE PATENTSCHRIFT

(11) **EP 2 295 046 B1**
(45) Veröffentlichungstag und Bekanntmachung des Hinweises auf die Patenterteilung: **19.12.2012**
(21) Anmeldenummer: 09170223.3
(22) Anmeldetag: 14.09.2009
(51) Int. Cl.: A61K 9/70, A61K 31/4468

(54) **Transdermales therapeutisches System zur Verabreichung von Fentanyl oder einem Analogstoff davon**
Transdermal therapeutic system for application of fentanyl or an analogue material thereof
Système thérapeutique transdermique destiné à l'application de fentanyle ou d'une matière analogue

(43) Veröffentlichungstag der Anmeldung: 16.03.2011
(73) Patentinhaber: Acino AG, 83714 Miesbach (DE)
(72) Erfinder: Salman, Nouha, 81373, München (DE); Schurad, Björn, 81667, München (DE); Teutsch, Ingo, 81371 München (DE)
(74) Vertreter: Best, Michael

(56) Entgegenhaltungen:
- EP-A- 0 272 987
- WO-A-01/64149
- US-A1- 2008 175 890

## Beschreibung

Gegenstand der vorliegenden Anmeldung ist ein System zum transdermalen Verabreichen von Fentanyl oder einem Analogstoff davon für therapeutische Zwecke. Das TTS zeichnet sich durch einen niedrigen Wirkstoffgehalt und eine geringe Pflastergröße aus.

Fentanyl und dessen Analogstoffe, insbesondere Alfentanil, Carfentanil, Lofentanil, Remifentanil, Sufentanil, Trefentanil und ähnliche Verbindungen sind wirksame synthetische Opiate. Fentanyl und dessen Analogstoffe sind hochwirksam und werden schnell metabolisiert. Problematisch bei Fentanyl ist, dass es einen relativ engen therapeutischen Index besitzt. Bei Überschreiten der Grenzwerte treten unerwünschte Nebeneffekte auf, insbesondere eine Beeinträchtigung der Atmung, die - wenn nicht geeignete Gegenmaßnahmen ergriffen werden - zum Tod führen kann. Die Wirkstoffe sind relativ teuer und es besteht ein sehr hohes Missbrauchsrisiko. Deshalb müssen Fentanylpflaster einerseits eine sehr präzise gesteuerte Freisetzung des Wirkstoffs gewährleisten und andererseits soll das Produkt so geschaffen sein, dass der Wirkstoff hieraus nicht leicht zu Missbrauchszwecken entfernt werden kann.

Ein Transdermalpflaster ist typischerweise eine kleine klebende Bandage, welche den abzugebenden Wirkstoff enthält. Diese Bandagen können verschiedene Formen und Größen haben. Der einfachste Typ ist ein Klebe-Monolith, welcher einen Wirkstoffvorrat (Reservoir) auf einem Träger umfasst. Das Reservoir wird typischerweise aus dem Wirkstoff in einem pharmazeutisch akzeptablen druckempfindlichen Klebstoff gebildet. Es kann aber auch aus einem nicht klebenden Material geformt sein, dessen Haut-Kontaktfläche mit einer dünnen Schicht eines geeigneten Klebstoffes versehen ist.

Komplexere Pflaster sind Mehrfachlaminate oder Pflaster mit Wirkstoffvorrat (der gegebenenfalls in einer Flüssigkeit gelöst vorliegen kann), in welchen zwischen dem Reservoir und dem die Haut kontaktierenden Klebstoff eine die Wirkstoff-Freisetzung steuernde Membran angeordnet sein kann. Diese Membran dient dazu, die Auswirkungen von Variationen der Hautdurchlässigkeit durch Herabsetzung der in vitro- und in vivo-Abgaberate des Wirkstoffs aus dem Pflaster zu kontrollieren und gegebenenfalls zu vermindern.

Das Reservoir der Transdermalpflaster kann den Wirkstoff entweder vollständig gelöst im Vorrat enthalten oder er kann einen Überschuss ungelösten Wirkstoffs enthalten (Depot-Pflaster). Die Anwesenheit von ungelöstem Wirkstoff oder anderen Bestandteilen in einem Pflaster kann bei der Lagerung sowie beim Gebrauch allerdings Stabilitäts- und andere Probleme aufwerfen. Eine Schwierigkeit besteht auch darin, dass sichergestellt werden muss, dass sich der Wirkstoff aus dem festen Depot ausreichend schnell nachlöst, um den abgegebenen Wirkstoff zu ersetzen. Wirkstoffpflaster, deren Reservoir feste Wirkstoffteilchen aufweist, werden im Stand der Technik daher häufig als nachteilig angesehen.

Aus dem Stand der Technik sind viele verschiedene transdermale Pflaster für die Verabreichung von Fentanyl bekannt. Die WO 02/074286 beschreibt ein transdermales Pflaster mit einem Reservoir enthaltend Fentanyl, wobei das Reservoir eine polymere Zusammensetzung, bevorzugt Polyacrylat, in einheitlichem Phasenzustand aufweist, die frei ist von nicht gelöstem Wirkstoff. Auch eine Übersättigung soll hier ausdrücklich vermieden werden.

Es gibt viele Versuche, Fentanylpflaster auch auf der Basis einer Matrixschicht aus Polyisobutylen herzustellen. Erste derartige Versuche werden bereits in dem Grundpatent zu Fentanylpflastern, dem US-A 4,588,580, beschrieben. Diese Druckschrift offenbart ein transdermales therapeutisches System mit einer Polyisobutylenmatrix und Mineralöl, die eine 2%ige Beladung von Fentanyl als ungelöstem Feststoff enthält. Das System weist in der Praxis jedoch Nachteile auf, und in der Folgezeit ging die Entwicklung daher von Polyisobutylenmatrices weg bzw. falls Polyisobutylenmatrices verwendet wurden, versuchte man, den Wirkstoff vollständig in der Polyisobutylenmatrix zu lösen.

Ein transdermales therapeutisches System mit einer Polyisobutylenmatrix ist in Roy et al., Journal of Pharmaceutical Sciences, Vol. 85, Nr. 5, Mai 1996, Seiten 491 bis 495 beschrieben. Es wird gezeigt, dass bei Konzentrationen von Fentanyl in der Polyisobutylenmatrix von mehr als 4% Wirkstoff ausfällt, und dies wurde offensichtlich von Roy et al. als negativ angesehen. Roy et al. schlägt für Pflaster mit geringer Wirkstoffbeladung an Fentanyl Polysilikonpflaster vor und keine Polyisobutylenpflaster.

In der EP 1 625 854, der US 2007/0009588 und der US 2006/0013865 werden dementsprechend Polyisobutylenmatrices vorgeschlagen, bei denen sorgfältig darauf zu achten ist, dass der Wirkstoff in der Polyisobutylenmatrix vollständig gelöst vorliegt. Das Auftreten von Kristallen in der Matrix wird als negativ angesehen.

Die DE 198 37 902 offenbart transdermale therapeutische Systeme auf der Basis von . Polyisobutylen, die insbesondere zur Verabreichung von Clonidin geeignet sind, unter den dort genannten Wirkstoffen findet sich aber auch Fentanyl. Beispiele für Fentanylpflaster finden sich in dieser Druckschrift nicht, ein Hinweis darauf, dass bei den dort offenbarten Pflastern der Wirkstoff als Feststoff vorhanden sein soll, findet sich in der Druckschrift ebenfalls nicht. Die Druckschrift enthält keine in vivo-Untersuchungen zur Freisetzung des Wirkstoffs aus den Pflastern. Die Polyisobutylenschicht dieser Pflaster enthält mindestens 5 Gew.-% eines Füllstoffs.

In der nicht vorveröffentlichten europäischen Patentanmeldung mit der Anmeldenummer 08155167.3 werden transdermale therapeutische Systeme zur Verabreichung von Fentanyl oder einem Analogstoff davon beschrieben, bei denen auf einer Rückschicht eine Polyisobutylenschicht aufgebracht ist, die den Wirkstoff enthält und die einen Gehalt an Gelbildner von höchstens 4 Gew.-% aufweist. Bei den dort beschriebenen transdermalen therapeutischen Systemen kann auf der Polyisobutylenschicht noch eine Klebstoffschicht aufgebracht sein, diese Klebstoffschicht enthält aber kein Fentanyl, und die Zusammensetzung der Klebstoffschicht ist nicht spezifiziert. Mit diesen Pflastern soll eine längere und gleichmäßigere Freisetzung erzielt werden, als mit den bekannten Fentanylpflastern.

Die US 2008/0175890 A1 betrifft Sufentanylpflaster, die darauf ausgelegt sind, einen möglichst hohen Fluss von Sufentanyl durch die Haut zur Verfügung zu stellen, ohne dass Penetrationsverstärker verwendet werden. Die dort offenbarten Pflaster auf Grundlage von PIB enthalten weder Weichmacher, noch Gelbildner. Die für ein kommerzielles Pflaster vorteilhaften Eigenschaften, wie gute Klebkraft in Verbindung mit einer langanhaltenden, langsamen Freisetzung des Wirkstoffs bei einem sehr kleinen Pflaster, weisen die dort offenbarten Pflaster nicht auf.

EP 0 272 987 A2 offenbart ein elastisches transdermales therapeutisches System enthaltend Fentanyl und ein Polyisobutylen.

WO 01/64149 A1 offenbart ein transdermales therapeutisches System enthaltend Fentanyl.

Es gibt derzeit noch kein Marktprodukt, bei dem es sich um ein Fentanylpflaster auf der Basis von Polyisobutylen handelt.

Die bekannten im Markt befindlichen Fentanylpflaster sind in der Regel Matrixpflaster auf Basis von Polyacrylaten, alle haben einen hohen Wirkstoffgehalt und weisen eine verhältnismäßig große Fläche von nicht unter 8 cm² auf. Eine größere Fläche wird bei der Applikation auf die Haut des Patienten als Nachteil angesehen. Aufgrund der hohen Preise der Wirkstoffe und des Missbrauchpotentials stellt es sich daher als vorteilhaft dar Pflaster zu entwickeln, die einerseits einen geringen Wirkstoffgehalt aufweisen und andererseits nach der Anwendung einen möglichst geringen Wirkstoffrestgehalt aufweisen.

Beispiele für marktübliche Pflaster von Fentanyl sind DUROGESIC^{®} SMAT, welches 4,2 mg Wirkstoff bei einer Pflastergröße von 10,5 cm² aufweist. Weniger Wirkstoff und eine geringere Pflastergröße weist das Marktprodukt MATRIFEN^{®} auf, ein Pflaster auf Polysilikonbasis, welches einen Wirkstoffgehalt von 2,75 mg bei einer Pflastergröße von 8,4 cm² aufweist. Es besteht aber Bedarf nach einem Pflaster, welches einen möglichst noch geringeren Wirkstoffgehalt bei gleicher oder sogar geringerer Pflastergröße aufweist, als diese bekannten Pflaster. Die Pflaster sollen eine möglichst gute Klebkraft aufweisen, also über die vorgesehene Anwendungsdauer von in der Regel drei Tagen oder auch mehr, auf der Haut haften, trotzdem leicht und ohne Schmerzen wieder entfernbar sein. Darüber hinaus sollen sie die gleiche oder zumindest eine möglichst ähnliche Blutplasmaspiegelkurve zur Verfügung stellen, wie die bekannten Marktprodukte und insbesondere das Marktprodukt DUROGESIC^{®} SMAT. Insbesondere sollen sie bevorzugt zu dem Produkt DUROGESIC^{®} SMAT im Wesentlichen bioäquivalent sein, wobei zur Definition des Begriffs "Bioäquivalenz" auf die WO 02/074286 verwiesen wird, auf deren diesbezügliche Offenbarung ausdrücklich Bezug genommen wird.

Im Hinblick auf den Stand der Technik stellt sich insbesondere die Aufgabe, ein transdermales therapeutisches System zur Verabreichung von Fentanyl oder eines Analogstoffs davon durch die Haut zur Verfügung zu stellen, das die Probleme des Standes der Technik nicht zeigt. Das Pflaster soll insbesondere in der Lage sein, Fentanyl bzw. einen Analogstoff davon gleichmäßig über einen langen Zeitraum in der zur Schmerzbekämpfung erforderlichen Menge freizusetzen. Der Blutplasmaspiegel sollte über einen möglichst langen Zeitraum, bevorzugt aber über einen Zeitraum von etwa 30 Stunden nach der Verabreichung bis etwa 72 Stunden nach der Verabreichung, insoweit konstant bleiben, dass er im Wesentlichen dem Blutplasmaspiegel entspricht, den das Marktprodukt DUROGESIC^{®} SMAT erreicht. Bevorzugte Ausführungsformen sind solche, bei denen das Pflaster zur Verabreichung alle drei Tage, alle vier Tage, alle fünf Tage, alle sechs Tage oder alle sieben Tage ausgestaltet ist. Bevorzugt sollte der Blutplasmaspiegel daher solange im Wesentlichen konstant bleiben, dass sich beispielsweise ein Dreitagepflaster, ein Viertagepflaster, ein Fünftagepflaster, ein Sechstagepflaster oder ein Siebentagepflaster ergibt. Besonders bevorzugt ist erfindungsgemäß ein Dreitagepflaster, entsprechend der Verabreichungszeit des Produkts DUROGESIC^{®} SMAT.

Zudem soll der Wirkstoffgehalt (bei gleicher Abgaberate) verglichen mit marktüblichen Pflastern des entsprechenden Wirkstoffs deutlich verringert sein, und das Pflaster soll eine geringere Fläche aufweisen als marktübliche Pflaster des entsprechenden Wirkstoffs wiederum selbstverständlich bei gleicher Abgaberate).

Gegenstand der vorliegenden Erfindung ist daher ein transdermales therapeutisches System zur Verabreichung eines Wirkstoffs durch die Haut, umfassend:
a) eine Rückschicht
b) ein auf der Rückschicht befindliches Reservoir, umfassend
   b1) eine erste Schicht, enthaltend Wirkstoff, zumindest einen Gelbildner, zumindest einen Weichmacher und ein erstes Polyisobutylengemisch und
   b2) eine zweite Schicht, enthaltend Wirkstoff, zumindest einen Gelbildner, zumindest einen Weichmacher und ein zweites Polyisobutylengemisch,
   wobei das erste Polyisobutylengemisch von dem zweiten Polyisobutylengemisch verschieden ist,
   wobei zumindest die erste Schicht ungelösten Wirkstoff in Form von Wirkstoffpartikeln enthält und
   wobei der Wirkstoff Fentanyl oder ein Analogstoff des Fentanyls und zwar Alfentanil, Lofentanil, Remifentanil oder Sufentanil ist.

Besonders bevorzugt handelt es sich bei dem Wirkstoff um Fentanyl oder Sufentanil, insbesondere um Fentanyl. Die Erfindung wird im Folgenden im Wesentlichen anhand des Fentanyls erläutert. Die Ausführungen gelten aber entsprechend auch für die Analogstoffe des Fentanyls.

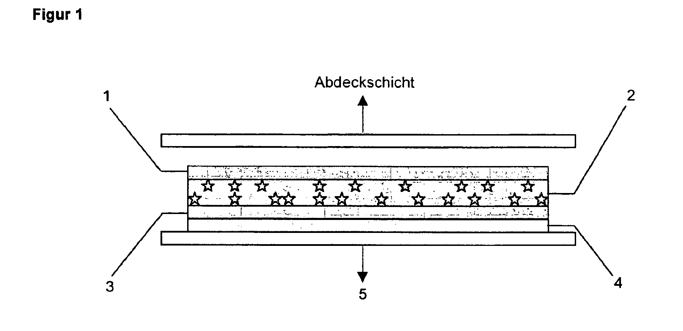

Der Aufbau eines bevorzugten transdermalen therapeutischen Systems im Querschnitt ist in Figur 1 dargestellt. In dem bevorzugten transdermalen therapeutischen System der Figur 1 besteht das transdermale therapeutische System aus einer Rückschicht 1, einem Reservoir, das aus einer ersten Schicht 2 und einer zweiten Schicht 4 besteht, sowie einer Abziehschicht 5. Das Pflaster kann ebenfalls noch eine Membran 3 enthalten (optional), die aber bevorzugt nicht vorhanden ist. Ferner kann auch eine Abdeckschicht vorhanden sein, die im Wesentlichen lose auf der Rückschicht aufliegt und beispielsweise durch elektrostatische Kräfte auf dieser haftet. Die Abdeckschicht ist bekannt und dient der leichteren Entnahme des Pflasters aus einer Verpackung. Sie ist nicht mit der Abziehschicht 5 zu verwechseln. Sowohl die Membran 3 als auch die Abdeckschicht und die Abziehschicht 5 sind optional, wobei in der Regel die Abziehschicht 5 bei dem Pflaster vorhanden sein wird. In Figur 1 symbolisieren die Sterne in der ersten Schicht 2 den in dieser Schicht vorliegenden ungelösten Wirkstoff.

Dieser Aufbau des transdermalen therapeutischen Systems ist erfindungsgemäß am stärksten bevorzugt. Es sind aber auch andere Ausführungsformen möglich, bei denen neben der ersten Schicht und der zweiten Schicht noch weitere Schichten (z.B. Klebstoffschichten ohne Wirkstoff, weitere Reservoirschichten, etc.) vorgesehen sind. Diese Ausführungsformen werden im Folgenden nicht im Einzelnen beschrieben, können von einem Fachmann aber ohne Weiteres aufgrund der Offenbarung der vorliegenden Anmeldung hergestellt werden. Die folgenden Ausführungsformen beziehen sich daher alle auf die bevorzugte Ausführungsform des erfindungsgemäßen transdermalen therapeutischen Systems mit genau zwei wirkstoffhaltigen Schichten und gegebenenfalls einer Membran, bei der keine weiteren Schichten vorhanden sind. Die Ausführungen gelten aber sinngemäß ebenfalls für weitere, nicht dargestellte Ausführungsformen mit weiteren Schichten, die gegebenenfalls auch noch Wirkstoff enthalten können.

Auf dem bei der Anwendung der Haut gegenüber befindlichen Ende des Pflasters befindet sich die Rückschicht 1. An der der menschlichen Haut beim Gebrauch zugewandten Seite der Rückschicht 1 befindet sich das Reservoir, umfassend eine erste Schicht 2 und eine zweite Schicht 4. Die in Kontakt mit der Rückschicht 1 befindliche erste Schicht 2 weist bevorzugt einen höheren Wirkstoffgehalt auf als die der Haut zugewandte zweite Schicht 4. Erfindungsgemäß bevorzugt ist die Klebkraft der zweiten Schicht 4 höher als die Klebkraft der ersten Schicht 2, so dass die zweite Schicht auch die Funktion einer Klebstoffschicht übernimmt.

Zumindest in der ersten Schicht 2 befindet sich ungelöster Wirkstoff in Form von Wirkstoffteilchen, auf die im Folgenden noch näher eingegangen wird.

Wenn im Rahmen der vorliegenden Anmeldung auf die Klebkraft Bezug genommen wird, erfolgt die Klebkraftmessung nach der entsprechenden DIN-Vorschrift. Soweit es erfindungsgemäß nicht auf die absolute Klebkraft der Schichten ankommt, sondern nur auf die relative Klebkraft der ersten Schicht zur zweiten Schicht, kann auch eine andere übliche Methode zur Bestimmung der Klebkraft verwendet werden, die von den DIN-Verfahren abweicht, sofern die Klebkraft der ersten Schicht und der zweiten Schicht nach der gleichen Methode bestimmt werden.

Auf der zweiten Schicht befindet sich bevorzugt noch eine Abziehschicht 5, die vor Gebrauch des transdermalen therapeutischen Systems abgezogen wird.

Die erste und die zweite Schicht weisen bei dem Pflaster bevorzugt die gleiche Fläche auf, so dass keine der Schichten über die andere hinausragt.

In einer weiteren Ausführungsform ist bei dem transdermalen therapeutischen System zwischen der ersten und der zweiten Schicht zusätzlich eine Membran 3 vorgesehen, die die Freisetzung des Wirkstoffs kontrolliert. Hauptzweck der Membran ist es, die in vivo- und in vitro-Freisetzungsrate des Wirkstoffs aus dem Pflaster zu reduzieren. Dadurch können Unterschiede in der Permeabilität für den Wirkstoff durch die Haut ausgeglichen werden. Bevorzugt handelt es sich bei der Membran um eine mikroporöse Membran.

Geeignete Membranen sind im Stand der Technik bekannt. In einer bevorzugten Ausführungsform kann die Membran Polypropylen oder Polyethylenvinylacetat enthalten oder daraus bestehen. Ein besonders bevorzugtes Material für die Membran ist eine mikroporöse Polypropylenfolie.

Die Dicke der Membran ist nicht besonders eingeschränkt und kann z.B. im Bereich von 10 µm bis 100 µm, bevorzugt kleiner 50 µm, z.B. bei etwa 25 µm, liegen. Die Porengröße liegt bevorzugt im Bereich von 0,001 bis 0,025 µm², z.B. im Bereich von 0,002 bis 0,011 µm², insbesondere bei etwa 0,005 µm². Die Form der Poren ist ebenfalls nicht besonders eingeschränkt, bevorzugt ist eine rechteckige Form.

Ein typisches Beispiel für eine geeignete Membran ist daher eine mikroporöse Polypropylenfolie mit einer Dicke von etwa 25 µm und einer Porengröße von etwa 0, 12 µm x 0,04 µm, wie sie unter dem Handelsnamen Celgard 2400 der Firma Celgard LLC, Charlotte, USA vertrieben wird.

Bevorzugt weist das erfindungsgemäße Pflaster keine Membran 3 auf.

Auf der zweiten Schicht befindet sich eine Abziehschicht (release liner), die in Figur 1 mit der Nummer 5 gekennzeichnet wurde. Diese Abziehschicht ist bevorzugt aus polymerem Material hergestellt, das gegebenenfalls auch metallisiert sein kann. Beispiele für bevorzugt eingesetzte polymere Materialien sind Polyurethane, Polyvinylacetat, Polyvinylidenchlorid, Polypropylen, Polycarbonat, Polystyrol, Polyethylen, Polyethylenterephthalat, Polybutylenterephthalat sowie gegebenenfalls mit entsprechenden Polymeren oberflächenbeschichtetes Papier. Bevorzugt handelt es sich hierbei um eine einseitig oder beidseitig fluorierte oder silikonisierte Abziehschicht. Besonders bevorzugt sind handelsübliche fluorierte oder silikonisierte Polyesterfolien, wie die einseitig silikonisierten Handelsprodukte Primeliner 100 µm, Perlosic LF 75 µm (Firma Loparex, NL und Perlen Converting AG, Schweiz), stärker bevorzugt sind die einseitig fluorierten Handelsprodukte Scotchpak 1022 oder Scotchpak 9742 (Firma 3M).

Erfindungsgemäß ist wesentlich, dass sowohl die erste Schicht als auch die zweite Schicht den Wirkstoff, bevorzugt Fentanyl, enthalten. Erfindungsgemäß bevorzugt ist die Konzentration des Wirkstoffs in der ersten Schicht höher als in der zweiten Schicht. Erfindungsgemäß liegt ein Teil des Fentanyls in der ersten Schicht in ungelöster Form vor, so dass die Konzentration des gelösten Fentanyls in der ersten Schicht der Sättigungslöslichkeit des Fentanyls in der ersten Schicht entspricht, die Gesamtkonzentration an Fentanyl in der ersten Schicht (gelöst + ungelöst) aber oberhalb der Sättigungslöslichkeit des Fentanyls in der ersten Schicht liegt.

Bevorzugt ist die Gesamtkonzentration des Fentanyls in der ersten Schicht (gelöster Bestandteil des Fentanyls und ungelöster Bestandteil des Fentanyls) im Bereich von 5 Gew.-% bis 15 Gew.-%, bevorzugt im Bereich von 8 Gew.-% bis 12 Gew.-% und stärker bevorzugt im Bereich von 9 Gew.-% bis 11 Gew.-%. Die Einheit Gewichtsprozent bezieht sich hier auf das Gesamtgewicht der ersten Schicht einschließlich des Wirkstoffs und aller anderen Bestanteile.

In der zweiten Schicht ist die Konzentration des Fentanyls bevorzugt unterhalb der Sättigungslöslichkeit des Fentanyls in der zweiten Schicht, so dass das gesamte Fentanyl in der zweiten Schicht bevorzugt in gelöster Form vorliegt. Es ist erfindungsgemäß aber auch möglich, dass in beiden Schichten, sowohl der ersten Schicht als auch in der zweiten Schicht, das Fentanyl teilweise in Form von Kristallen vorliegt.

Die Konzentration des Fentanyls in der zweiten Schicht beträgt bevorzugt 0,5 Gew.-% bis 3 Gew.-%, stärker bevorzugt 1 bis 2,5 Gew.-% und insbesondere 1 Gew.-% bis 2 Gew.-%. Die vorstehenden Angaben beziehen sich auf das Gesamtgewicht der zweiten Schicht einschließlich des Wirkstoffs und aller übrigen Bestandteile.

Soweit in der vorliegenden Anmeldung auf die Sättigungslöslichkeit bzw. die Sättigungskonzentration von Fentanyl Bezug genommen wird, beziehen sich diese Angaben auf eine Temperatur von 25°C, und die Sättigungslöslichkeit wird wie folgt bestimmt: Es werden eine Reihe von Pflastern mit steigenden Fentanylkonzentrationen hergestellt und bei 25°C gelagert. Die Pflaster werden über einen Zeitraum von sechs Monaten beobachtet. Die höchste Konzentration, bei der nach sechs Monaten noch kein Wirkstoff auskristallisiert ist (bzw. als Feststoff ausgefallen ist), stellt die untere Grenze der Sättigungslöslichkeit dar. Die niedrigste Konzentration, bei der nach sechs Monaten Wirkstoff auskristallisiert ist (bzw. als Feststoff ausgefallen ist), stellt die obere Grenze der Sättigungslöslichkeit dar. Die Sättigungslöslichkeit liegt dann innerhalb des durch die beiden Grenzwerte gebildeten Bereichs. Die Genauigkeit der Messmethode kann erhöht werden, indem die Abstände zwischen den Konzentrationen verringert werden. Die Beobachtung erfolgt optisch (mit dem Auge).

Die Sättigungslöslichkeit des Fentanyls in den beiden Schichten kann auf an sich bekannte Art und Weise eingestellt werden, z.B. indem den Schichten Stoffe zugesetzt werden, die die Sättigungslöslichkeit beeinflussen, oder durch die Wahl entsprechender Matrixpolymere. Erfindungsgemäß besonders bevorzugt erfolgt die Einstellung der Sättigungslöslichkeit durch Variation des Weichmachers bzw. der Konzentration des Weichmachers in der Schicht.

Es ist erfindungsgemäß bevorzugt, dass die Fentanylkonzentration in der zweiten Schicht geringer ist als die Fentanylkonzentration in der ersten Schicht. Bevorzugt ist das Verhältnis von Fentanyl in der ersten Schicht zu Fentanyl in der zweiten Schicht (Gewichtsbasis) im Bereich von 4 bis 12, stärker bevorzugt im Bereich von 6 bis 10 und insbesondere im Bereich von 7 bis 9.

Die Konzentration des Wirkstoffs im gesamten Reservoir, also sowohl in der ersten Schicht als auch in der zweiten Schicht und gegebenenfalls weiteren wirkstoffhaltigen Schichten des Reservoirs beträgt bevorzugt 3 Gew.-% bis 20 Gew.-%, stärker bevorzugt 4 Gew.-% bis 15 Gew.-%, insbesondere 5 Gew.-% bis 10 Gew.-%, wobei sich die Gewichtsprozentangabe auf das Gesamtgewicht aller wirkstoffhaltigen Schichten in dem Reservoir einschließlich des Wirkstoffs und aller anderen Bestandteile der Schichten bezieht.

Es ergeben sich bevorzugt Flächengewichte im Bereich von 20 bis 100 g/m², stärker bevorzugt 25 bis 80 g/m², insbesondere im Bereich von 30 bis 70 g/m². Das Reservoir weist bevorzugt eine Dicke (Trockendicke) im Bereich von 20 bis 400 µm, stärker bevorzugt im Bereich von 30 bis 200 µm, insbesondere im Bereich von 40 bis 100 µm auf (alle Schichten einschließlich der gegebenenfalls vorhandenen Membran).

Es versteht sich von selbst, dass bei den erfindungsgemäßen transdermalen therapeutischen Systemen alle wirkstoffhaltigen Schichten Bestandteil des Reservoirs sind, sich also außerhalb des Reservoirs keine wirkstoffhaltigen Schichten befinden.

Bei dem Weichmacher handelt es sich um eine prinzipiell bekannte Verbindung, die im Stand der Technik in transdermalen therapeutischen Systemen als Weichmacher eingesetzt wird. Der Weichmacher dient bevorzugt auch noch dazu, die Penetration des Wirkstoffs durch die Haut zu verbessern, und in einer besonders bevorzugten Ausführungsform reguliert er die Löslichkeit des Wirkstoffs in dem Reservoir, so dass ein bestimmter Gehalt an Wirkstoff in Lösung gehalten wird. Der Weichmacher befindet sich daher in beiden Schichten, sowohl der ersten Schicht als auch der zweiten Schicht, wie vorstehend definiert. Es ist bevorzugt, dass der Weichmacher in beiden Schichten die gleiche Verbindung ist, es ist aber ebenfalls möglich, dass sich in der ersten Schicht ein anderer Weichmacher befindet als in der zweiten Schicht, insbesondere falls gewünscht wird, dass der Weichmacher die Löslichkeit des Wirkstoffs in der ersten Schicht anders einstellt als in der zweiten Schicht.

Bei dem Weichmacher handelt es sich bevorzugt um Mineralöl, Leinsamenöl, Octylpalmitat, Squalen, Squalan, Silikonöl, Isobutylmyristat, Isostearylalkohol oder Oleylalkohol, wobei Mineralöle als Weichmacher bevorzugt sind. Diese auch als dünnflüssige Paraffine bezeichneten Öle sind farblose, klare Kohlenwasserstoffe. Sie werden aus den oberhalb von etwa 300°C siedenden Destillationsfraktionen des Erdöls gewonnen und durch Abkühlen von festen Kohlenwasserstoffen befreit. Durch Extrahieren mit Lösungsmittel sowie durch Behandlung mit Bleicherden und/oder Schwefelsäure werden sie raffiniert. Geeignete Mineralöle sind sowohl in chemischer wie auch in biologischer Hinsicht stabil und verhindern bakterielles Wachstum. Durch geeignete Fraktionierung können Mineralöle gewonnen werden, die etwa bei Körpertemperatur, also bei etwa 35 bis 37°C flüssig sind, bei tieferen Temperaturen, insbesondere bei Temperaturen unter 20°C fest sind. Die Auswahl eines Mineralöls mit einem Verflüssigungspunkt von etwa 30-35°C ist bevorzugt.

Bevorzugt ist der prozentuale Gehalt an Weichmacher in der ersten Schicht höher als in der zweiten Schicht.

Der Weichmacher ist in der ersten Schicht bevorzugt in einer Menge im Bereich von 10 bis 60 Gew.-%, stärker bevorzugt von 25 bis 50 Gew.%, insbesondere im Bereich von 30 bis 40 Gew.%, vorhanden (bezogen auf das Gesamtgewicht der ersten Schicht).

Der Weichmacher ist in der zweiten Schicht bevorzugt in einer Menge im Bereich von 1 bis 15 Gew.-%, stärker bevorzugt von 2 bis 10 Gew.-%, insbesondere von 3 bis 8 Gew.-%, z.B. etwa 5 Gew.-% vorhanden (jeweils bezogen auf das Gesamtgewicht der zweiten Schicht).

In dem transdermalen therapeutischen System muss das Reservoir eine Menge an Fentanyl oder einem Analogstoff davon enthalten, die ausreichend ist um Schmerzfreiheit bei einem Menschen zu induzieren und für den gewünschten Zeitraum, bevorzugt für mindestens zwei Tage, stärker bevorzugt mindestens drei Tage, aufrechtzuerhalten (bezogen auf den Zeitpunkt der Verabreichung des Pflasters). Bevorzugt enthält das Reservoir eine Menge an Fentanyl oder Analogstoff davon, die ausreichend ist, um Schmerzfreiheit zu induzieren und für einen Zeitraum von mindestens drei Tagen, insbesondere von drei bis sieben Tagen, insbesondere von drei Tagen, aufrechtzuerhalten.

Die Schichten des erfindungsgemäßen Pflasters beinhalten auch einen Gelbildner. Hierbei handelt es sich bevorzugt um einen Gelbildner mit partikulärer Struktur, der auf seiner Oberfläche eine hohe Konzentration polarer Gruppen aufweist. Diese verursachen zu den Ölen (Weichmacher) hin entsprechend hohe Grenzflächenspannungen, die durch Agglomeration der Partikel untereinander zu Gelgerüsten teilweise kompensiert werden. Demzufolge sind die Gelgerüste immer um so fester, je größer der Polaritätsunterschied zwischen den Ölen und der Gelbildneroberfläche ist. Bevorzugt wird erfindungsgemäß als Gelbildner hochdisperses Siliziumdioxid oder kolloidales Siliziumdioxid eingesetzt. Die Größe der Partikel bewegt sich bevorzugt im Nanobereich und liegt z.B. im Bereich von 400 bis 1500 nm, insbesondere im Bereich von 500 bis 1000 nm. Kolloidales Siliziumdioxid wird beispielsweise unter der Bezeichnung Cab-O-Sil^{®} vertrieben und ist ein bekanntes Verdickungsmittel für Mineralöl. Ein anderes Beispiel für einen geeigneten Gelbildner ist Bentonit. Auch das als Gelbildner bekannte Natriumcarbomer kann verwendet werden. Eingesetzt wird der Gelbildner in jeder Schicht in einer Menge von bevorzugt 0,1 bis 4,0, stärker bevorzugt 0,5 bis 2,0 Gew.-% bezogen auf das Gesamtgewicht der jeweiligen Schicht.

Wie bei dem Weichmacher ausgeführt, ist es auch bei dem Gelbildner bevorzugt, dass jede Schicht, insbesondere die erste und die zweite Schicht, den gleichen Geldbildner aufweist. Es ist aber auch möglich, dass in jeder Schicht ein unterschiedlicher Gelbildner eingesetzt wird.

Erfindungsgemäß ist wesentlich, dass das Polyisobutylengemisch der ersten Schicht (erstes Polyisobutylengemisch) von den Polyisobutylenen der zweiten Schicht (zweites Polyisobutylengemisch) verschieden ist. Erfindungsgemäß bevorzugt hat das erste Polyisobutylengemisch ein anderes mittleres Molekulargewicht als das zweite Polyisobutylengemisch (im Rahmen dieser Anmeldung wird immer auf das gewichtsgemittelte Molekulargewicht M_{w} Bezug genommen, sofern nichts anderes erwähnt oder aufgrund des Zusammenhangs offensichtlich ist; das gewichtsgemittelte Molekulargewicht M_{w} wird in der Regel über GPC bestimmt, wie es dem Fachmann bekannt ist).

Erfindungsgemäß bevorzugt ist das mittlere Molekulargewicht des ersten Polyisobutylengemischs im Bereich von 100.000 bis 10.000.000 und das mittlere Molekulargewicht des zweiten Polyisobutylengemischs im Bereich von 15.000 bis 5.000.000, wobei das mittlere Molekulargewicht des zweiten Polyisobutylengemischs niedriger ist, als das mittlere Molekulargewicht des ersten Polyisobutylengemischs.

Erfindungsgemäß bevorzugt besteht das erste Polyisobutylen aus einem Gemisch aus mindestens zwei Polyisobutylenen mit unterschiedlichem mittleren Molekulargewicht. Dies bedeutet, dass die Molekulargewichtsverteilung des ersten Polyisobutylens mindestens zwei Peaks bei unterschiedlichen Molekulargewichten aufweist.

Erfindungsgemäß bevorzugt besteht das zweite Polyisobutylen aus einem Gemisch aus mindestens zwei Polyisobutylenen mit unterschiedlichem mittleren Molekulargewicht. Dies bedeutet, dass die Molekulargewichtsverteilung des zweiten Polyisobutylens ebenfalls mindestens zwei Peaks bei unterschiedlichen Molekulargewichten aufweist.

Erfindungsgemäß besonders bevorzugt besteht sowohl das erste als auch das zweite Polyisobutylen aus einem Gemisch aus mindestens zwei, stärker bevorzugt aus zwei Polyisobutylenen, eines mit höherem mittleren Molekulargewicht, eines mit niedrigerem mittleren Molekulargewicht.

Bevorzugt hat das eine Polyisobutylen der Gemische (höhermolekulares Polyisobutylen) ein mittleres Molekulargewicht von 150.000 bis 10.000.000, besonders bevorzugt von 500.000 bis 10.000.000, und das andere Polyisobutylen der Gemische hat ein geringeres mittleres Molekulargewicht (geringermolekulares Polyisobutylen) im Bereich von 15.000 bis 100.000, vorzugsweise von 20.000 bis 80.000. Vor allem das Polyisobutylen mit dem niedrigeren mittleren Molekulargewicht ist verantwortlich für die Klebrigkeit des Pflasters.

In einer Ausführungsform der vorliegenden Erfindung ist das mittlere Molekulargewicht des höhermolekularen Polyisobutylens in dem ersten Polyisobutylengemisch von dem mittleren Molekulargewicht des höhermolekularen Polyisobutylens in dem zweiten Polyisobutylengemisch verschieden und/oder das mittlere Molekulargewicht des geringermolekularen Polyisobutylens in dem Gemisch des ersten Polyisobutylengemisch ist von dem mittleren Molekulargewicht des geringermolekularen Polyisobutylens in dem zweiten Polyisobutylengemisch verschieden.

Erfindungsgemäß bevorzugt ist aber das mittlere Molekulargewicht des höhermolekularen Polyisobutylens in dem ersten Polyisobutylengemisch und dem zweiten Polyisobutylengemisch im Wesentlichen gleich. Erfindungsgemäß ebenfalls bevorzugt, ist auch das mittlere Molekulargewicht des geringermolekularen Polyisobutylens in dem ersten Polyisobutylengemisch und in dem zweiten Polyisobutylengemisch im Wesentlichen gleich. Das erste Polyisobutylengemisch unterschiedet sich dann von dem zweiten Polyisobutylengemisch dadurch, dass das Verhältnis der beiden Polyisobutylene (höhermolekulares Polyisobutylen : geringermolekulares Polyisobutylen) in dem ersten Polyisobutylengemisch anders ist als in dem zweiten Polyisobutylengemisch.

Das heißt, der Anteil des Polyisobutylens mit hohem Molekulargewicht ist in dem ersten Polyisobutylengemisch ein anderer als in dem zweiten Polyisobutylengemisch, und entsprechend ist der Anteil des Polyisobutylens mit geringerem Molekulargewicht in dem ersten Polyisobutylengemisch ein anderer als in dem zweiten Polyisobutylengemisch.

Erfindungsgemäß bedeutet "im Wesentlichen gleich", dass sich die entsprechenden Werte (beispielsweise des mittleren Molekulargewichts) um nicht mehr als 10%, bezogen auf den höchsten Wert, unterscheiden. Bevorzugt wird darunter verstanden, dass die Werte im Rahmen der Messgenauigkeit gleich sind.

Bevorzugt ist in dem ersten Polyisobutylengemisch das Verhältnis von dem Polyisobutylen mit höherem Molekulargewicht zu dem Polyisobutylen mit geringerem Molekulargewicht im Bereich von 0,05:1 bis 20:1, besonders bevorzugt von 0,5:1 bis 2:1, insbesondere im Bereich von etwa 1:1.

In dem zweiten Polyisobutylengemisch ist in der Regel der Anteil an Polyisobutylen mit geringerem Molekulargewicht höher als in dem ersten Polyisobutylengemisch, und insbesondere ist das Verhältnis von Polyisobutylen mit geringerem Molekulargewicht zu dem Polyisobutylen mit höherem Molekulargewicht hier bevorzugt im Bereich von 1:1 bis 30:1, insbesondere im Bereich von 2:1 bis 15:1, beispielweise bei etwa 9:1, das heißt dass etwa 9 mal soviel Polyisobutylen mit geringerem Molekulargewicht als Polyisobutylen mit höherem Molekulargewicht vorhanden ist (alle Angaben beziehen sich auf das Gewicht).

Bezogen auf das Gesamtgewicht der ersten Schicht beträgt der Anteil des ersten Polyisobutylengemischs bevorzugt 30 bis 80 Gew.-%, stärker bevorzugt 40 bis 65 Gew.-%, insbesondere 50 bis 60 Gew.-%.

Bezogen auf das Gesamtgewicht der zweiten Schicht beträgt der Anteil des zweiten Polyisobutylengemischs bevorzugt 50 bis 98 Gew.-%, stärker bevorzugt 60 bis 95 Gew.-%, insbesondere 80 bis 95 Gew.-%, z.B. etwa 93 Gew.-%.

Polyisobutylene mit bestimmtem gewichtsgemitteltem Molekulargewicht sind handelsüblich und können als erstes bzw. zweites Polyisobutylengemisch gemäß der Erfindung verwendet werden. Die erfindungsgemäß besonders bevorzugten Gemische aus mindestens zwei Polyisobutylenen mit unterschiedlichem Molekulargewicht können z.B. durch Mischen der handelsüblichen Polyisobutylene hergestellt werden. Hierzu werden beide handelsüblichen Polyisobutylene in einem geeigneten Lösemittel, z.B. n-Heptan, gelöst und miteinander gemischt. Anschließend kann das Lösemittel geeignet entfernt werden.

Beispiele für Polyisobutylene mit höherem Molekulargewicht sind die kommerziellen Produkte Oppanol B80, B100, B150 und B200, bevorzugt Oppanol B80 oder B100, insbesondere Oppanol B100 (M_{w} = 1.100.000), und Beispiele für Polyisobutylene mit niedrigerem Molekulargewicht sind die kommerziellen Produkte Oppanol B10 SFN bis B15 SFN, insbesondere Oppanol B10 SFN (M_{w} = 36.000). Ein Beispiel für ein bevorzugtes erstes Polyisobutylengemisch ist ein Gemisch aus Oppanol B100 und Oppanol B10 SFN im Verhältnis von 1:1, und ein Beispiel für ein bevorzugtes zweites Polyisobutylengemisch ist ein Gemisch aus Oppanol B100 und Oppanol B10 SFN im Verhältnis 1:9.

Sofern im Rahmen dieser Beschreibung von einem "Gesamtgewicht" gesprochen wird, ist hierunter immer das Trockengewicht einschließlich aller Bestandteile, also das Gewicht in dem anwendungsfertigen Pflaster, zu verstehen, sofern nichts anderes offenbart oder ersichtlich ist.

Wesentlich für die erfindungsgemäßen transdermalen therapeutischen Systeme ist, dass zumindest die erste Schicht so viel Wirkstoff enthält, dass ein Teil des Wirkstoffs in ungelöster Form, also als Wirkstoffpartikel, vorliegt.

Bei der Herstellung des erfindungsgemäßen Pflasters wird der Wirkstoff bevorzugt in mikronisierter Form mit einer mittleren Teilchengröße von 50 µm oder weniger, bevorzugt mit einer mittleren Teilchengröße von 20 µm oder weniger, eingesetzt. Auch in der ersten Schicht des Pflasters liegt der Wirkstoff in mikronisierter Form vor, allerdings können sich durch Umlagerungsreaktionen bei der Lagerung des Pflasters geringfügige Abweichungen der Teilchengröße ergeben. Auch innerhalb des Pflasters beträgt die mittlere Teilchengröße der Wirkstoffpartikel aber bevorzugt weniger als 100 µm, stärker bevorzugt weniger als 50 µm und insbesondere etwa 20 µm oder darunter. Zur Herstellung des erfindungsgemäßen Pflasters wird bevorzugt mikronisiertes Fentanyl mit einer mittleren Teilchengröße von 1 µm oder mehr, stärker bevorzugt 2 µm oder mehr, eingesetzt. Diese mittleren Teilchengrößen finden sich bevorzugt ebenfalls in den fertig hergestellten Pflastern. In den Schichten des transdermalen therapeutischen Systems kann die Teilchengröße und die Teilchengrößenverteilung der Wirkstoffteilchen am besten durch übliche Lichtmikroskopie bestimmt werden. Die Auswertung erfolgt über übliche Computerprogramme (Bildverarbeitungssysteme), die in der Regel auf die verwendeten Mikroskope abgestimmt sind. Die Teilchengröße bezieht sich auf den Teilchendurchmesser, sofern nichts anderes angegeben oder offensichtlich ist.

Als Ausgangsmaterial für das mikronisierte Fentanyl wird handelsübliches Fentanyl eingesetzt, das an sich für die klinische Anwendung geeignet ist. Derartiges Fentanyl hat üblicherweise eine Verteilung der Partikelgröße dergestalt, dass 100% der Partikel kleiner sind als 2500 µm. Etwa 90% der Partikel sind kleiner als etwa 1000 µm und 50% der Partikel sind kleiner als etwa 100 µm.

Erfindungsgemäß kann jedes bekannte Mikronisierungsverfahren verwendet werden, das die gewünschte Teilchengröße zur Verfügung stellt. Bevorzugt wird Fentanyl eingesetzt, das mittels einer üblichen "jet mill" mikronisiert wurde, z.B. einer jet mill vom Typ AS der Hosokawa Alpine AG.

Durch das erfindungsgemäß eingesetzte Mikronisierungsverfahren wird die Größe der Fentanylpartikel bevorzugt so eingestellt, dass die mittlere Teilchengröße in den vorstehend angegebenen Bereichen liegt. Bevorzugt ist ebenfalls, dass 100% der Partikel kleiner sind als 50 µm, insbesondere als 20 µm. Etwa 90% der mikronisierten Partikel sind bevorzugt kleiner als 12 µm und etwa 50% der Partikel sind bevorzugt kleiner als 6 µm.

Für die Bestimmung der Teilchengröße bzw. der Teilchengrößenverteilung des Wirkstoffs gibt es verschiedene Verfahren, beispielsweise Lichtbeugungsverfahren (Laserdiffraktometrie), wie sie in den Geräten der Firma Malvern Instruments, z.B. dem "Malvern MasterSizer X", verwendet werden, mechanische Siebrüttelverfahren, wie sie die Firma FMC zur Bestimmung der Korngrößenverteilung ihrer AVICEL PH^{®}-Produkte verwendet, oder auch "air jet"-Siebanalysen, die beispielsweise mit einem ALPINA^{®}-"air jet" Modell 200 ausgeführt werden können.

Sofern nicht anders angegeben, werden die (mittleren) Teilchengrößen bzw. Teilchengrößenverteilungen mit dem Verfahren Laserdiffraktometrie bestimmt, beispielweise mit dem Gerät Mastersizer 2000 von Malvern.

Definiert man den Wirkstoff über die Angabe der mittleren Teilchengröße und die Teilchengrößenverteilung, weist der erfindungsgemäß eingesetzte mikronisierte Wirkstoff bevorzugt eine mittlere Teilchengröße von 20 µm oder weniger auf, und es ist bevorzugt, dass der Wirkstoff eine Korngrößenverteilung (Teilchengrößenverteilung) hat, bei der weniger als 10% der Teilchen eine Größe von 25 µm oder darüber haben und weniger als 10% der Teilchen eine Größe von 1 µm oder darunter haben. Noch weiter bevorzugt ist ein Wirkstoff mit einer mittleren Teilchengröße von 15 µm. Bevorzugt hat ein derartiger Wirkstoff eine Korngrößenverteilung, bei der weniger als 2% der Teilchen eine Größe von 20 µm oder darüber haben und weniger als 50% der Teilchen eine Größe von 6 µm oder darunter haben. Die Korngrößenverteilung für den Wirkstoff sollte so eng wie möglich sein.

Auf der der menschlichen Haut beim Gebrauch abgewandten Seite des Reservoirs befindet sich eine Rückschicht, die in bevorzugter Ausführungsform okklusiv, also abschließend ist. Derartige Rückschichten können in bevorzugter Ausführungsform aus Polyolefinen, insbesondere Polyethylen, oder aus Polyester sowie Polyurethanen bestehen. Auch Schichten, die mehrere verschiedene Polymere übereinander angeordnet beinhalten, sind vorteilhafterweise einsetzbar. Ein besonders bevorzugtes Material für die Rückschicht ist ein Polyolefin, das von der Firma Mylan Technologies Inc. unter der Bezeichnung Mediflex^{®}1000 vertrieben wird. Andere geeignete Materialien umfassen Cellophan, Celluloseacetat, Ethylcellulose, mit Weichmachern versehene Vinylacetatvinylchloridcopolymere, Ethylenvinylacetatcopolymere, Polyethylenterephthalat, Nylon, Polyethylen, Polypropylen, Polyvinylidenchlorid, Ethylenmethacrylatcopolymer, Papier, das gegebenenfalls beschichtet sein kann, textile Gewebe, Polyesterfolien, wie Polyethylentherephthalatfolien. Besonders bevorzugt sind Aluminiumfolie und Polymer-Metall-Kompositmaterialien. Die Dicke der Rückschicht ist, wie im Stand der Technik üblich, z.B. 10 µm bis 80 µm, z.B. etwa 55 µm nominelle Dicke.

Auf der Rückschicht des Pflasters befindet sich bevorzugt eine Abdeckschicht, die insbesondere verhindern soll, dass das Pflaster mit der Verpackung verklebt, falls geringe Mengen Polyisobutylen oder Klebstoff austreten. Die Abdeckschicht liegt bevorzugt lose auf der Rückschicht auf und wird durch elektrostatische Kräfte gehalten. Derartige Abdeckschichten sind im Stand der Technik bekannt, z.B. aus der EP 1 097 090, auf die insoweit vollinhaltlich Bezug genommen wird. Die Abdeckschicht ist zumindest auf der auf der Rückschicht aufliegenden Seite antihaftbeschichtet, z.B. silikonisiert oder fluoriert.

Die Herstellung eines bevorzugten transdermalen therapeutischen Systems erfolgt, indem man zunächst die Bestandteile für die erste Schicht mischt, z.B. zunächst Fentanyl und den Gelbildner, in einem organischen Medium wie Heptan dispergiert (bzw. Teile des Fentanyls löst) und das Mineralöl und Polyisobutylen in einem organischen Medium, bevorzugt dem gleichen wie vorher, miteinander mischt. Anschließend werden Fentanyl und Gelbildner in dem Gemisch aus Polyisobutylen und Mineralöl dispergiert. Bei der Herstellung dieses Gemischs wird bevorzugt ein flüchtiges organisches Medium, wie beispielsweise Heptan, eingesetzt. Diese Mischung wird dann als gleichmäßige Schicht auf der Rückschicht aufgebracht und getrocknet. Falls eine Membran aufgebracht werden soll, wird diese nun auf der der Rückschicht gegenüberliegenden Seite des Reservoirs aufgebracht.

In einem separaten Arbeitsschritt werden die Bestandteile der zweiten Schicht im Wesentlichen auf gleiche Art und Weist miteinander gemischt, wie es vorstehend für die erste Schicht beschrieben ist. Allerdings ist bei der zweiten Schicht bevorzugt, dass das gesamte Fentanyl in Lösung gebracht wird. Die einzusetzende Menge an Fentanyl sollte also bevorzugt unterhalb der Sättigungslöslichkeit des Fentanyls in der zweiten Schicht liegen, und bei der Herstellung ist darauf zu achten, dass sich das gesamte Fentanyl löst. Das Gemisch mit den Bestandteilen der zweiten Schicht und dem Lösemittel wird dann auf die Abziehfolie aufgebracht und getrocknet.

Die in den beiden Verfahrensschritten erhaltenen Komponenten werden anschließend miteinander laminiert, und zwar so, dass die zweite Schicht auf die Membran aufgebracht wird, falls eine derartige Membran vorgesehen ist. In denjenigen Ausführungsformen, in denen keine Membran eingesetzt wird, wird die zweite Schicht direkt auf die erste Schicht laminiert. Anschließend können aus der fertigen laminierten Folie Stücke gewünschter Größe ausgestanzt werden und verpackt werden.

Bei den einzelnen Verfahrensschritten werden die organischen Lösungsmittel, die erforderlich sind, um das Polyisobutylen in Lösung zu bringen und die anderen Bestandteile zu dispergieren oder zu lösen, in der Regel dadurch entfernt, dass die Produkte ansteigenden Temperaturen, gegebenenfalls auch unter Anwendung eines Unterdrucks, ausgesetzt werden.

Mit den erfindungsgemäßen transdermalen therapeutischen Systemen ist es möglich, praxisgerechte Pflaster zur Verabreichung von Fentanyl oder einem Analogstoff davon zur Verfügung zu stellen, die gegenüber den bekannten Pflastern, insbesondere gegenüber den kommerziell erhältlichen Pflastern, weniger Wirkstoff enthalten und eine kleinere Pflasterfläche aufweisen. Insbesondere bei der Verabreichung des Wirkstoffs Fentanyl kann ein Pflaster zur Verfügung gestellt werden, das im Wesentlichen zu den Durogesic-Produkten bioäquivalent ist (hierzu siehe die Definition in der WO 02/074286), aber erheblich weniger Wirkstoff enthält und kleiner ist. So enthält das DUROGESIC^{®} SMAT-Pflaster, das eine Abgaberate von 25 µg/h liefert, 4,2 mg Fentanyl, und die Pflastergröße beträgt 10,5 cm². Demgegenüber enthält ein erfindungsgemäßes Pflaster, das ebenfalls eine Abgaberate von 25 µg/h liefert und zu dem DUROGESIC^{®} SMAT-Pflaster im Wesentlichen bioäquivalent ist, nur 2,23 mg Fentanyl, und die Pflastergröße ist nur 6,6 cm². Diese Werte sind sogar besser als die Werte der derzeitig auf dem Markt befindlichen Polysilikonpflaster, wie die des Produkts MATRIFEN^{®}, das bei einer Abgaberate von 25 µg/h nur 2,75 mg Fentanyl und eine Pflastergröße von 8,4 cm² aufweist.

Die folgenden Beispiele erläutern die Erfindung, sind aber nicht einschränkend.

### Beispiel 1 (Vergleichsbeispiel)

### a) Herstellung der ersten Schicht

Für die Herstellung eines transdermalen Pflasters wurde zunächst Fentanyl mit einer mittleren Partikelgröße von 20 µm (z.B. Gesellschaft für Mikronisierung mbH, jet mill, AS, Hosokawa Alpine AG) in Heptan mit dem Polyisobutylen (Gemisch Oppanol B100: Oppanol B10 SFN 1:1, gelöst in n-Heptan), Siliziumdioxid (Cab-O-Sil M-5P) als Gelbildner und Mineralöl "Klearol" als Weichmacher dispergiert. Die Mischung wurde als dünne Schicht auf die Rückschicht aufgetragen und trocknen gelassen, so dass sich ein Flächengewicht von etwa 55 g/m² ergab.

### b) Anbringen der Membran

Hierauf wurde als Membran ein mikroporöser Polypropylen-Film (Celgard 2400) aufgebracht.

### c) Herstellung der zweiten Schicht

Parallel hierzu wurde nur Polyisobutylen (Gemisch Oppanol B100: Oppanol B10 SFN 1:1, gelöst in Heptan) in dünner Schicht auf die Abziehschicht aufgebracht und trocknen gelassen, so dass sich ein Flächengewicht von etwa 30 g/m² ergab. Die Toleranz der Flächengewichte betrug 10%.

### d) Herstellung des Pflasters

Nachdem beide Schichten getrocknet waren, wurden die beiden Schichten miteinander laminiert, wobei die Membran mit dem Reservoir durch Anwendung geeigneten Drucks verbunden wurde.

Anschließend wurden rechteckige Pflaster mit abgerundeten Kanten in einer Größe von 10 cm² ausgestanzt und mit der Abdeckfolie verpackt (Patchgröße 10 cm²). Das fertige Produkt wies die folgende Zusammensetzung auf:

| **Name des Bestandteils** | **Menge** | **Konzentration (%) des anwendungsfertigen (trockenen) Reservoirs, je Schicht** |
|---|---|---|
| **Erste Schicht** | | |
| Fentanyl | 4,95 mg | 9% |
| Gemisch Oppanol B100: | 30,0 mg | 54,5% |
| Oppanol B10 SFN 1:1 | | |
| Cab-O-Sil M-5P | 0,55 mg | 1% |
| Klearol (Mineralöl) | 19,5 mg | 35,5% |
| Heptan* | --- | --- |

| **Zweite Schicht** | | |
|---|---|---|
| Gemisch Oppanol B100: | 30,0 mg | 100% |
| Oppanol B10 SFN 1:1 | | |
| Heptan* | --- | --- |

| **Schichtmaterialien** | | |
|---|---|---|
| Celgard 2400 | 10,0 cm² | |
| Hostaphan MN 19 MED | 10,0 cm² | |
| Primeliner 100 µm PET | 15 cm² | |
| C1S | | |
| PETP Film, 36 µm, | 15 cm² | |
| transparent | | |
| Aluminiumverpackung, | 1 Beutel | |
| weiß | | |

| | | |
|---|---|---|
| * im fertigen Produkt nicht vorhanden | | |

Die in dem Beispiel eingesetzten Komponenten können näher beschrieben werden wie folgt:

| **Komponentenbezeichnung** | **chemische Beschreibung** | **Funktion** |
|---|---|---|
| Gemisch Oppanol B100: | Polyisobutylenklebemittel | Haftklebstoff |
| Oppanol B10 SFN 1:1 | gelöst in n-Heptan | |
| Cab-O-Sil M-5P | kolloidales Siliziumdioxid | gelbildendes Mittel |
| Klearol | leichtes Mineralöl | Weichmacher |
| Celgard 2400 | mikroporöser Polypropylenfilm | Membran |
| Hostaphan MN 19 MED | Polyethylenterephthalatfilm mit 19 µm nomineller Dicke | Rückschicht |
| Primeliner 100 µm PET C1S | Polyesterfilm mit 100 µm Nominaldicke, eine Seite silikonisiert | Abziehschicht |
| PETP-Film, 36 µm, | Polyesterfilm mit nominaler | Abdeckschicht |
| transparent | Dicke, 36 µm, eine Seite silikonisiert | |
| Aluminiumpackverpackung, | Heißklebeverpackung | Verpackungsmaterial |
| weiß | | |

### Beispiel 2 (erfindungsgemäß)

Auf gleiche Art und Weise wie in Beispiel 1 (Vergleichsbeispiel) beschrieben, wurde unter Verwendung der Bestandteile wie in der folgenden Tabelle angegeben mit den in der Tabelle angegebenen Konzentrationen ein transdermales therapeutisches System hergestellt. Allerdings wurde auf die erste Schicht keine Membran aufgebracht, und bei der Herstellung der zweiten Schicht wurde darauf geachtet, dass alles Fentanyl vollständig aufgelöst war. Dementsprechend wurde Schritt b) des Beispiels 1 (Vergleichsbeispiel) nicht durchgeführt, und bei Schritt d) wurden die beiden Schichten miteinander laminiert, ohne dass die erste Schicht mit einer Membran bedeckt war.

Anschließend wurden rechteckige Pflaster mit abgerundeten Kanten in einer Größe von etwa 7 cm² ausgestanzt und mit der Abdeckfolie verpackt (nominelle Freisetzung 25 µg/h, Patchgröße 6,6 cm²).

| **Name des Bestandteils** | **Menge** | **Konzentration (%) des anwendungsfertigen (trockenen) Reservoirs, je Schicht** |
|---|---|---|
| **Erste Schicht** | | |
| Fentanyl | 1,98 mg | 10% |
| Gemisch Oppanol B100: Oppanol B10 SFN 1:1 | 10.59 mg | 53,5% |
| n-Heptan* | - | - |
| Siliziumdioxid (z.B. Cab- O-Sil M-5P) | 0,20 mg | 1% |
| leichtes Mineralöl (z.B. Klearol) | 7,03 mg | 35,5% |

| **Zweite Schicht** | | |
|---|---|---|
| Fentanyl | 0,25 mg | 1,5% |
| Siliziumdioxid (z.B. Cab- O-Sil M-5P) | 0,08 mg | 0,5% |
| leichtes Mineralöl (z.B. Klearol) | 0,83 mg | 5% |
| Oppanol B10 SFN | 13,81 mg | 83,7% |
| Oppanol B100 | 1,54 mg | 9,3% |
| n-Heptan* | - | - |

| **Schichtmaterialien** | | |
|---|---|---|
| Scotchpak 9738 | 6,6 cm² | |
| Scotchpak 9742 | 11 cm² | |
| PETP Film, 36 µm, transparent | 11 cm² | |
| Aluminiumverpackung, weiß | 1 Beutel | |

| | | |
|---|---|---|
| * im fertigen Produkt nicht vorhanden | | |

## Patentansprüche

1. Transdermales therapeutisches System zur Verabreichung eines Wirkstoffs durch die Haut, umfassend:
a) eine Rückschicht
b) ein auf der Rückschicht befindliches Reservoir, umfassend
b1) eine erste Schicht, enthaltend Wirkstoff, zumindest einen Gelbildner, zumindest einen Weichmacher und ein erstes Polyisobutylengemisch und
b2) eine zweite Schicht, enthaltend Wirkstoff, zumindest einen Gelbildner, zumindest einen Weichmacher und ein zweites Polyisobutylengemisch,
wobei das erste Polyisobutylengemisch von dem zweiten Polyisobutylengemisch verschieden ist,
wobei zumindest die erste Schicht ungelösten Wirkstoff in Form von Wirkstoffpartikeln enthält und
wobei der Wirkstoff Fentanyl, Alfentanil, Lofentanil, Remifentanil oder Sufentanil ist.

2. Transdermales therapeutisches System nach Anspruch 1, wobei das erste Polyisobutylengemisch ein Gemisch aus einem Polyisobutylen mit einem höheren gewichtsgemittelten Molekulargewicht und aus einem Polyisobutylen mit einem niedrigeren gewichtsgemittelten Molekulargewicht ist.

3. Transdermales therapeutisches System nach Anspruch 1 oder 2, wobei das zweite Polyisobutylengemisch ein Gemisch aus einem Polyisobutylen mit einem höheren gewichtsgemittelten Molekulargewicht und aus einem Polyisobutylen mit einem niedrigeren gewichtsgemittelten Molekulargewicht ist.

4. Transdermales therapeutisches System nach Anspruch 3, wobei das Polyisobutylen mit höherem gewichtsgemitteltem Molekulargewicht in dem ersten Polyisobutylengemisch im Wesentlichen das gleiche gewichtsgemittelte Molekulargewicht hat, wie das Polyisobutylen mit höherem gewichtsgemitteltem Molekulargewicht in dem zweiten Polyisobutylengemisch und das Polyisobutylen mit niedrigerem gewichtsgemitteltem Molekulargewicht in dem ersten Polyisobutylengemisch im Wesentlichen das gleiche gewichtsgemittelte Molekulargewicht hat, wie das Polyisobutylen mit niedrigem gewichtsgemitteltem Molekulargewicht in dem zweiten Polyisobutylengemisch.

5. Transdermales therapeutisches System nach einem der Ansprüche 2 bis 4, wobei das Polyisobutylen mit niedrigerem gewichtsgemitteltem Molekulargewicht ein gewichtsgemitteltes Molekulargewicht im Bereich von 15.000 bis 100.000 aufweist und das Polyisobutylen mit höherem gewichtsgemitteltem Molekulargewicht ein gewichtsgemitteltes Molekulargewicht im Bereich von 500.000 bis 10.000.000 aufweist.

6. Transdermales therapeutisches System nach einem der Ansprüche 1 bis 5, **dadurch gekennzeichnet, dass** die Konzentration des Wirkstoffs im Reservoir im Bereich von 3 Gew.-% bis 20 Gew.-% liegt, bezogen auf das Gewicht sämtlicher wirkstoffhaltiger Schichten.

7. Transdermales therapeutisches System nach einem der Ansprüche 1 bis 6, bei dem die erste Schicht auf der Rückschicht aufgebracht ist und den Wirkstoff in einer höheren Konzentration enthält als die zweite Schicht.

8. Transdermales therapeutisches System nach einem der Ansprüche 1 bis 7, das weiterhin eine Abziehschicht aufweist.

9. Transdermales therapeutisches System nach einem der Ansprüche 1 bis 8, bei dem entweder die zweite Schicht auf der ersten Schicht aufgebracht ist oder eine Membran auf der ersten Schicht aufgebracht ist und die zweite Schicht auf der Membran aufgebracht ist.

10. Transdermales therapeutisches System nach einem der Ansprüche 1 bis 9, wobei die zweite Schicht eine höhere Klebkraft aufweist als die erste Schicht.

11. Transdermales therapeutisches System nach einem der Ansprüche 1 bis 10, **dadurch gekennzeichnet, dass** das Reservoir eine Menge an Fentanyl, Alfentanil, Lofentanil, Remifentanil oder Sufentanil enthält, die ausreichend ist, um bei einem menschlichen Patienten Schmerzfreiheit zu induzieren und für einen Zeitraum von drei bis sieben Tagen aufrecht zu erhalten.

12. Transdermales therapeutisches System nach einem der Ansprüche 1 bis 11, **dadurch gekennzeichnet, dass** es sich bei dem Weichmacher um Mineralöl handelt und dass es sich bei dem Gelbildner um kolloidales Siliziumdioxid oder um Bentonit handelt.

13. Transdermales therapeutisches System nach einem der Ansprüche 1 bis 12, **dadurch gekennzeichnet, dass** die Wirkstoffpartikel in dem Reservoir in mikronisierter Form mit einer mittleren Teilchengröße von 50 µm oder weniger, insbesondere von 20 µm oder weniger, vorliegen.

14. Transdermales therapeutisches System nach einem der Ansprüche 1 bis 13, **dadurch gekennzeichnet, dass** es sich bei der Rückschicht um eine okklusive Rückschicht handelt.

15. Transdermales therapeutisches System nach einem der Ansprüche 1 bis 14, **dadurch gekennzeichnet, dass** die erste Schicht zu 30 bis 80 Gew.-% und die zweite Schicht zu 50 bis 98 Gew.-% aus Polyisobutylen besteht, jeweils bezogen auf das Gesamtgewicht der jeweiligen Schicht.

16. Transdermales therapeutisches System nach einem der Ansprüche 1 bis 15, **dadurch gekennzeichnet, dass** der Weichmacher in der ersten Schicht in einer Konzentration von 10 bis 60 Gew.-% und in der zweiten Schicht in einer Konzentration von 1 bis 15 Gew.-% vorhanden ist, jeweils bezogen auf das Gesamtgewicht der jeweiligen Schicht.

17. Transdermales therapeutisches System nach einem der Ansprüche 1 bis 16, **dadurch gekennzeichnet, dass** die erste Schicht den Wirkstoff in einer Konzentration von 3 Gew.-% bis 15 Gew.-% enthält und die zweite Schicht den Wirkstoff in einer Konzentration von 0,5 Gew.-% bis 5 Gew.-% enthält, jeweils bezogen auf das Gesamtgewicht aller wirkstoffhaltigen Schichten, wobei die Konzentration des Wirkstoffs in der ersten Schicht höher ist als in der zweiten Schicht.

18. Verfahren zur Herstellung eines transdermalen therapeutischen Systems nach einem der Ansprüche 1 bis 17, wobei ein erstes Gemisch mit den Bestandteilen der ersten Schicht und gegebenenfalls einem oder mehreren Lösemitteln auf die Rückschicht des transdermalen therapeutischen Systems aufgebracht wird, gegebenenfalls Lösungsmittel entfernt wird, ein zweites Gemisch mit den Bestandteilen der zweiten Schicht und gegebenenfalls einem oder mehreren Lösemitteln auf eine Abziehschicht aufgebracht wird und gegebenenfalls Lösemittel entfernt wird, gegebenenfalls eine Membran auf die erste oder die zweite Schicht aufgebracht wird und die Schichten laminiert werden.

## Claims

1. A transdermal therapeutic system for administering an active ingredient through the skin comprising:
a) a back layer,
b) a reservoir on the back layer comprising
b1) a first layer containing active ingredient, at least one gelling agent, at least one plasticizer, and a first polyisobutylene mixture and
b2) a second layer containing active ingredient, at least one gelling agent, at least one plasticizer, and a second polyisobutylene mixture,
wherein the first polyisobutylene mixture is different from the second polyisobutylene mixture,
wherein at least the first layer contains undissolved active ingredient in the form of active ingredient particles and
wherein the active ingredient is Fentanyl, alfentanil, lofentanil, remifentanil or sufentanil.

2. The transdermal therapeutic system according to claim 1, wherein the first polyisobutylene mixture is a mixture of a polyisobutylene with a higher weight average molecular weight and a polyisobutylene with a lower weight average molecular weight.

3. The transdermal therapeutic system according to claim 1 or 2, wherein the second polyisobutylene mixture is a mixture of a polyisobutylene with a higher weight average molecular weight and a polyisobutylene with a lower weight average molecular weight.

4. The transdermal therapeutic system according to claim 3, wherein the polyisobutylene with the higher weight average molecular weight in the first polyisobutylene mixture has substantially the same weight average molecular weight as the polyisobutylene with the higher weight average molecular weight in the second polyisobutylene mixture and the polyisobutylene with the lower weight average molecular weight in the first polyisobutylene mixture has substantially the same weight average molecular weight as the polyisobutylene with the lower weight average molecular weight in the second polyisobutylene mixture.

5. The transdermal therapeutic system according to any one of claims 2 to 4, wherein the polyisobutylene with the lower weight average molecular weight has a weight average molecular weight in the range of 15,000 to 100,000 and the polyisobutylene with the higher weight average molecular weight has a weight average molecular weight in the range of 500,000 to 10,000,000.

6. The transdermal therapeutic system according to any one of claims 1 to 5, **characterized in that** the concentration of the active ingredient in the reservoir is in the range of 3% by weight to 20% by weight, based on the weight of all active ingredient-containing layers.

7. The transdermal therapeutic system according to any one of claims 1 to 6, wherein the first layer is applied onto the back layer and contains the active ingredient in a higher concentration than the second layer.

8. The transdermal therapeutic system according to any one of claims 1 to 7 further having a release liner.

9. The transdermal therapeutic system according to any one of claims 1 to 8, wherein either the second layer is applied onto the first layer or a membrane is applied onto the first layer and the second layer is applied onto the membrane.

10. The transdermal therapeutic system according to any one of claims 1 to 9, wherein the second layer has a higher adhesiveness than the first layer.

11. The transdermal therapeutic system according to any one of claims 1 to 10, **characterized in that** the reservoir contains an amount of Fentanyl, alfentanil, lofentanil, remifentanil or sufentanil sufficient to induce analgesia in a human patient and to maintain it for a period of three to seven days.

12. The transdermal therapeutic system according to any one of claims 1 to 11, **characterized in that** the plasticizer is a mineral oil and that the gelling agent is colloidal silica or bentonite.

13. The transdermal therapeutic system according to any one of claims 1 to 12, **characterized in that** the active ingredient particles in the reservoir are present in a micronized form with an average particle size of 50 µm or less, in particular of 20 µm or less.

14. The transdermal therapeutic system according to any one of claims 1 to 13, **characterized in that** the back layer is an occlusive back layer.

15. The transdermal therapeutic system according to any one of claims 1 to 14, **characterized in that** the first layer consists of 30 to 80% by weight and the second layer of 50 to 98% by weight of polyisobutylene, each based on the total weight of the respective layer.

16. The transdermal therapeutic system according to any one of claims 1 to 15, **characterized in that** the plasticizer in the first layer is present in a concentration of 10 to 60% by weight and in the second layer in a concentration of 1 to 15% by weight, each based on the total weight of the respective layer.

17. The transdermal therapeutic system according to any one of claims 1 to 16, **characterized in that** the first layer contains the active ingredient in a concentration of 3% by weight to 15% by weight and the second layer contains the active ingredient in a concentration of 0.5% by weight to 5% by weight, each based on the total weight of all active ingredient-containing layers, wherein the concentration of the active ingredient in the first layer is higher than in the second layer.

18. A method for the preparation of a transdermal therapeutic system according to any one of claims 1 to 17, wherein a first mixture with the constituents of the first layer and optionally one or more solvents is applied onto the back layer of the transdermal therapeutic system, optionally solvent is removed, a second mixture with the constituents of the second layer and optionally one or more solvents is applied onto the release liner and optionally solvent is removed, optionally a membrane is applied onto the first or the second layer and the layers are laminated.

## Revendications

1. Système thérapeutique transdermique destiné à l'administration d'un principe actif à travers la peau, comprenant :
a) une couche dorsale
b) un réservoir se trouvant sur la couche dorsale, comprenant
- b1) une première couche contenant du principe actif, au moins un filmogène, au moins un plastifiant et un premier mélange de polyisobutylène, et
- b2) une deuxième couche contenant du principe actif, au moins un filmogène, au moins un plastifiant et un deuxième mélange de polyisobutylène,
où le premier mélange de polyisobutylène est différent du deuxième mélange de polyisobutylène,
où au moins la première couche contient du principe actif non dissous sous la forme de particules de principe actif et
où le principe actif est le fentanyle, l'alfentanil, le lofentanil, le rémifentanil ou le sufentanil.

2. Système thérapeutique transdermique selon la revendication 1, où le premier mélange de polyisobutylène est un mélange constitué d'un polyisobutylène ayant un poids moléculaire moyen en poids élevé et d'un polyisobutylène ayant un poids moléculaire moyen en poids faible.

3. Système thérapeutique transdermique selon la revendication 1 ou 2, où le deuxième mélange de polyisobutylène est un mélange constitué d'un polyisobutylène ayant un poids moléculaire moyen en poids élevé et d'un polyisobutylène ayant un poids moléculaire moyen en poids faible.

4. Système thérapeutique transdermique selon la revendication 3, où le polyisobutylène ayant un poids moléculaire moyen en poids élevé dans le premier mélange de polyisobutylène a sensiblement le même poids moléculaire moyen en poids que le polyisobutylène ayant un poids moléculaire moyen en poids élevé dans le deuxième mélange de polyisobutylène et le polyisobutylène ayant un poids moléculaire moyen en poids faible dans le premier mélange de polyisobutylène a sensiblement le même poids moléculaire moyen en poids que le polyisobutylène ayant un poids moléculaire moyen en poids faible dans le deuxième mélange de polyisobutylène.

5. Système thérapeutique transdermique selon l'une des revendications 2 à 4, où le polyisobutylène ayant un poids moléculaire moyen en poids faible présente un poids moléculaire moyen en poids situé dans la plage allant de 15 000 à 100 000 et le polyisobutylène ayant un poids moléculaire moyen en poids élevé présente un poids moléculaire moyen en poids situé dans la plage allant de 500 000 à 10 000 000.

6. Système thérapeutique transdermique selon l'une des revendications 1 à 5, **caractérisé en ce que** la concentration du principe actif dans le réservoir se situe dans la plage allant de 3 % en poids à 20 % en poids, par rapport au poids de la totalité des couches contenant du principe actif.

7. Système thérapeutique transdermique selon l'une des revendications 1 à 6, dans lequel la première couche est déposée sur la couche dorsale et contient le principe actif en une concentration supérieure à la deuxième couche.

8. Système thérapeutique transdermique selon l'une des revendications 1 à 7, qui présente en plus une couche pelliculable.

9. Système thérapeutique transdermique selon l'une des revendications 1 à 8, dans lequel soit la deuxième couche est déposée sur la première couche soit une membrane est déposée sur la première couche et la deuxième couche est déposée sur la membrane.

10. Système thérapeutique transdermique selon l'une des revendications 1 à 9, où la deuxième couche présente une force d'adhésion supérieure à la première couche.

11. Système thérapeutique transdermique selon l'une des revendications 1 à 10, **caractérisé en ce que** le réservoir contient une quantité de fentanyle, d'alfentanil, de lofentanil, de rémifentanil ou de sufentanil, qui est suffisante pour induire une absence de douleur chez un patient humain et la maintenir pendant une durée allant de trois à sept jours.

12. Système thérapeutique transdermique selon l'une des revendications 1 à 11, **caractérisé en ce que** le plastifiant est une huile minérale et le filmogène est le dioxyde de silicium colloïdal ou la bentonite.

13. Système thérapeutique transdermique selon l'une des revendications 1 à 12, **caractérisé en ce que** les particules de principe actif dans le réservoir se présentent sous forme micronisée ayant une taille de particules moyenne de 50 µm ou moins, en particulier de 20 µm ou moins.

14. Système thérapeutique transdermique selon l'une des revendications 1 à 13, **caractérisé en ce que** la couche dorsale est une couche dorsale occlusive.

15. Système thérapeutique transdermique selon l'une des revendications 1 à 14, **caractérisé en ce que** la première couche se compose de 30 à 80 % en poids et la deuxième couche se compose de 50 à 98 % en poids de polyisobutylène, à chaque fois rapporté au poids total de la couche en question.

16. Système thérapeutique transdermique selon l'une des revendications 1 à 15, **caractérisé en ce que** le plastifiant dans la première couche est présent en une concentration de 10 à 60 % en poids et dans la deuxième couche en une concentration de 1 à 15 % en poids, à chaque fois rapporté au poids total de la couche en question.

17. Système thérapeutique transdermique selon l'une des revendications 1 à 16, **caractérisé en ce que** la première couche contient le principe actif en une concentration de 3 à 15 % en poids et la deuxième couche contient le principe actif en une concentration de 0,5 à 5 % en poids, à chaque fois rapporté au poids total de toutes les couches contenant du principe actif, où la concentration du principe actif dans la première couche est supérieure à celle de la deuxième couche.

18. Procédé de production d'un système thérapeutique transdermique selon l'une des revendications 1 à 17, où un premier mélange avec les composants de la première couche et le cas échéant avec un ou plusieurs solvants est déposé sur la couche dorsale du système thérapeutique transdermique, le solvant est retiré le cas échéant, un deuxième mélange avec les composants de la deuxième couche et le cas échéant un ou plusieurs solvants est déposé sur une couche pelliculable et le solvant est retiré le cas échéant, une membrane est déposée le cas échéant sur la première ou la deuxième couche et les couches sont stratifiées.
